(19) Europäisches Patentamt
European Patent Office
Office européen des brevets

(11) Veröffentlichungsnummer: **0 096 292**
**B1**

(12) # EUROPÄISCHE PATENTSCHRIFT

(45) Veröffentlichungstag der Patentschrift: **23.07.86**

(51) Int. Cl.⁴: **C 07 H 15/04**, C 07 H 3/06, A 61 K 39/00

(21) Anmeldenummer: **83105146.1**

(22) Anmeldetag: **25.05.83**

(54) **Beta-D-Galactosederivate, Verfahren zu ihrer Herstellung und ihre Verwendung.**

(30) Priorität: **29.05.82 DE 3220427**

(43) Veröffentlichungstag der Anmeldung: **21.12.83 Patentblatt 83/51**

(45) Bekanntmachung des Hinweises auf die Patenterteilung: **23.07.86 Patentblatt 86/30**

(84) Benannte Vertragsstaaten: **AT BE CH DE FR GB IT LI LU NL SE**

(56) Entgegenhaltungen:
**EP-A-0 060 999**

**TETRAHEDRON LETTERS, Band 22, Nr. 15, 1981, Pergamon Press Ltd., J. JACQUINET et al.: "Synthese von Oligosaccharid-Determinanten des T-Antigens mit Amid-Spacer zur Darstellung synthetischer Antigene 1)", Seiten 1387-1390**
**CARBOHYDRATE RESEARCH, Band 101, Nr. 2, März 1982, Elsevier Scientific Publ. Co., AMSTERDAM (NL), T. OGAWA et al.: "Synthesis of 3-0(2-Acetamido-2-Deoxy-3-0-beta-D-Galactopyrano syl-beta-beta-D-Galactopyranosyl)-1,2-Di-0-Tetradecyl-sn-Glycerol"**

(73) Patentinhaber: **BEHRINGWERKE Aktiengesellschaft, Postfach 1140, D-3550 Marburg 1 (DE)**

(72) Erfinder: **Kolar, Cenek, Dr., Lindenweg 14, D-3550 Marburg 1 (DE)**

(74) Vertreter: **Meyer- Dulheuer, Karl- Hermann, Dr., HOECHST Aktiengesellschaft Zentrale Patentabteilung Postfach 80 03 20, D-6230 Frankfurt/Main 80 (DE)**

**EP 0 096 292 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Verbindungen der allgemeinen Formel I

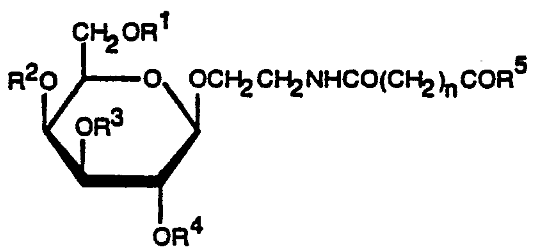

I

worin
$R^1$ und $R^2$ je ein Wasserstoffatom, eine Acyl- oder Benzyl-Schutzgruppe,
$R^1$ und $R^2$ zusammen eine Alkyliden- oder Benzyliden-Schutzgruppe
$R^4$ ein Wasserstoffatom, eine Acyl- oder Benzyl-Schutzgruppe oder einen 2,3,4 - Tri - O - benzyl - alpha - L - fucopyranosyl - oder alpha - L - fucopyranosyl - Rest,
$R^5$ H, OH, $NH_2$, $NHNH_2$, $N_3$, O-Alkyl, O-Aryl, NH—$(CH_2)_mNH_2$ mit m=2−5, Lysin; Polylysin oder einen Träger, n 1 bis 10 und
$R^3$ ein Wasserstoffatom, eine Acyl- oder Benzyl-Schutzgruppe oder einen alpha-D-Glycopyranosyl-Rest der allgemeinen Formel II bedeuten

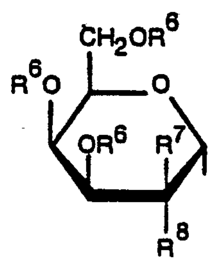

II

worin
$R^6$ ein Wasserstoffatom oder eine Acyl- oder Benzyl-Schutzgruppe,
$R^7$ ein Wasserstoffatom oder Halogen und
$R^8$ ein Wasserstoffatom, eine Acyloxy- oder Benzyloxy-Gruppe oder eine Azido-, Amino-, Acetamido- oder Hydroxy-Gruppe bedeuten,
sowie ein Verfahren zu ihrer Herstellung und ihre Verwendung, gebunden an einen Träger als künstliche Antigene, Glycolipide oder Immunadsorbentien.

Die Oberfläche menschlicher Erythrozyten ist in gleicher Weise wie die anderer Zelltypen mit einem Mosaik spezifischer Determinanten bedeckt, das aus einer großen Anzahl komplexer Oligosaccharid-Ketten besteht. Es ist bekannt (R. R. Race und R. Sänger, Blood Groups in Man, Blackwell Scientific Publ., Oxford 1975), daß Kohlenhydratverbindungen die Antigendeterminanten zahlreicher Stoffe sind. Oligosaccharide, die selbst keine Antigenwirkung aufweisen, besitzen antigene Eigenschaften, wenn sie an einen geeigneten hochmolekularen Trägerstoff gebunden sind.

Für die Herstellung künstlicher Antigene und Immunadsorbentien mit Strukturmerkmalen der ABO-Blutgruppen-aktiven Determinanten werden chemische Verbindungen mit diesen Strukturmerkmalen gebraucht, die mit geeigneten Trägermolekülen umgesetzt werden können.

Es ist bekannt, daß Kohlenhydrate, wenn sie über einen Spacer an einen Träger gebunden werden, zu künstlichen Antigenen werden.

Die vorliegende Erfindung hatte sich zur Aufgabe gestellt, Verbindungen herzustellen, aus denen künstliche ABO-Blutgruppen-aktive Antigene, Immunoadsorbentien und Glykolipide hergestellt werden könne, indem sie kovalent an Träger gebunden werden.

Gelöst wird diese Aufgabe durch die Herstellung von Verbindungen der allgemeinen Formel I und ihre Bindung an Träger.

Bevorzugt sind Verbindungen der Formel I, worin der Kohlenhydratrest ß-D-Galactopyranosyl oder
$R^1$ und $R^2$ Wasserstoffatome und
$R^4$ ein Wasserstoffatom oder alpha-L-fucopyranosyl und
$R^3$ Wasserstoff, N-Acetyl-alpha-D-galactosaminyl, alpha-D-Galactopyranosyl oder 2 - Desoxy - alpha - D - galactopyranosyl und
$R^5$ $OCH_3$, $NHNH_2$, $N_3$, NH—$CH_2CH_2NH_2$, Protein, besonders Albumin, ein $NH_2$-Gruppen tragendes Gel oder Kephalin und n=7 sind.

Das erfindungsgemäße Verfahren zur Herstellung einer der neuen Verbindungen der Formel I ist dadurch gekennzeichnet, daß man

a) eine Verbindung der allgemeinen Formel III

$HOCH_2CH_2NHCO(CH_2)_nCOR^5$ III

in der $R^5$ O-Alkyl oder O-Benzyl und n=1 bis 10 bedeuten, in an sich bekannter Weise mit einer Verbindung der allgemeinen Formel IV

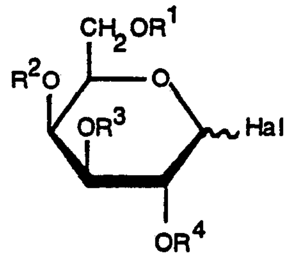

IV

in der $R^1$, $R^2$, $R^3$ und $R^4$ eine Acylgruppe, bevorzugt Acetyl- oder Benzoyl-Gruppe, und Hal Cl, Br oder J bedeuten, bevorzugt zu einem ß-Glycosid der Formel I umsetzt,

b) in dem Produkt von Stufe a) in an sich bekannter Weise die Acylgruppen alkalisch abspaltet und zu einer 4,6 - O - Alkyliden - oder 4,6 - Benzyliden - Verbindung der Formel I, worin

$R^3$ und $R^4$ je ein Wasserstoffatom und

$R^1$ und $R^2$ zusammen die Benzyliden- oder eine Alkylidengruppe bedeuten, umsetzt,

c) das Produkt von Stufe b) in an sich bekannter Weise zu einer Verbindung der allgemeinen Formel I, worin

$R^3$ eine Acylgruppe, bevorzugt Acetyl- oder Benzoylgruppe, bedeutet, umsetzt,

d) das Produkt aus Stufe c) in an sich bekannter Weise mit 2,3,4 - Tri - O - benzyl - alpha - L - fucopyranosylhalogenid zu einem alpha-verknüpften Disaccharid-Derivat der Formel I, worin

$R^2$, 2,3,4 - Tri - O - benzyl - alpha - L - fucopyranosyl ist, umsetzt, und gegebenenfalls zu einer weiteren Verbindung der Formel I entblockiert,

e) in dem blockierten Produkt aus Stufe d) in an sich bekannter Weise selektiv die 3 - O - Acylgruppe abspaltet, wodurch man eine Verbindung der Formel I erhält, worin $R^4$ 2,3,4 - Tri - O - benzyl - alpha - fucopyranosyl und $R^3$ Wasserstoff ist und

$R^1$, $R^2$ und $R^5$ die zur Formel IV genannte Bedeutung haben

f) das Produkt aus Stufe e) in an sich bekannter Weise mit einer Verbindung der allgemeinen Formel V

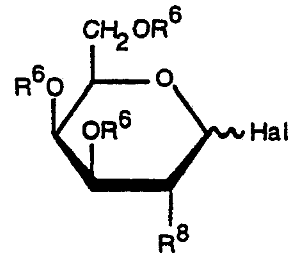

V

worin

$R^6$ eine Acylgruppe, bevorzugt Acetyl- oder Benzylgruppe,

$R^8$ die Azido- oder Benzyloxygruppe und Hal Br oder Cl ist, zu einem alpha-verknüpften Trisaccharid-Derivat der Formel I umsetzt, worin

$R^4$ 2,3,4 - Tri - O - benzyl - alpha - L - fucopyranosyl und

$R^3$ 3,4,6 - Tri - O - benzyl - 2 - azido - alpha - L - galactopyranosyl sind, und gegebenenfalls zu weiteren Verbindungen der Formeln VI und VII

α—D—GalNAc(1—3)—ß—D—Gal—O—$CH_2CH_2NHCO(CH_2)_nCOR^5$

2
1 ↑
α—L—Fuc VI

α—D—Gal(1—3)—ß—D—Gal—O—$CH_2CH_2NHCO(CH_2)_nCOR^5$

2
1 ↑
α—L—Fuc VII

entblockiert,

g) oder das Produkt aus Stufe e) in an sich bekannter Weise mit 3,4,6 - Tri - O - acetyl - D - galactal in Gegenwart von N-Jod- oder-Brom-succinimid zu einem alpha-verknüpften Trisaccharid-Derivat der Formel I, worin

$R^4$ 2,3,4 - Tri - O - benzyl - alpha - L - fucopyranosyl und

$R^3$ 3,4,6 - Tri - O - acetyl - 2 - brom - oder - jod - alpha - D - galactosyl sind, umsetzt und gegebenenfalls zu einer weiteren Verbindung der Formel VIII

2-desoxy-α-D—Gal(1—3)—ß—D—Gal—O—$CH_2CH_2NHCO(CH_2)_nCOR^5$

2
1 ↑
α—L—Fuc VIII

entblockiert, und

h) eine vorstehend erhaltene Verbindung der Formel I, worin $R^5$ O-Alkyl oder O-Aryl ist, in an sich bekannter Weise zu einer Verbindung der Formel I umsetzt, worin

$R^5$ OH, NH—$NH_2$, $N_3$, HN—$(CH_2)_m$—$NH_2$ mit m=2 bis 5, Lysin, Polylysin, NH-Protein, ein $NH_2$-Gruppen tragendes Gel oder NH-Kephalin bedeutet.

Die Produkte von Stufe h), worin $R^5$ eine Azidogruppe ist, können in an sich bekannter Weise an lösliche oder unlösliche Aminogruppen tragende Träger unter Bildung einer Saueramid-Gruppierung gebunden werden.

Solche Träger sind zum Beispiel Peptide und Proteine, bevorzugt Human- oder Rinderserum-Albumin oder Polylysin, aminierte Kunststoffe oder Gele, bevorzugt mit Ethylendiamin aktiviertes oder aminiertes Kieselgel, aminierte Polysaccharide oder Lipide, bevorzugt Kephaline oder aminierte Phospholipide.

Die Produkte von Stufe h), die eine NH—$(CH_2)_m$—$NH_2$-Gruppierung besitzen, können in an sich bekannter Weise über die primäre Aminogruppe an lösliche oder unlösliche Träger, die eine Oxiran-Gruppierung aufweisen, unter Bildung von sekundären Aminen gebunden werden.

Die an Träger gebundenen Verbindungen der Formel I, worin

$R^4$ alpha-L-fucopyranosyl und

$R^3$ Wasserstoff, N - Acetyl - alpha - D - galactosaminyl, alpha - D - Galactopyranosyl oder 2 - Desoxy - alpha - D - galactopyranosyl sind, stellen synthetische Antigene oder Immunadsorbentien dar.

Die folgenden Beispiele erläutern die Erfindung:

Beispiel 1

N - (8 - Methoxycarbonylcapryl) - 2 - aminoethyl - 2,3,4,6 - tetra - O - acetyl - beta - D - galactopyranosid (Verbindung 1)

20 g (81,5 mMol) N - (8 - Methoxycarbonylcapryl) - 2 - aminoethanol wurden in 1000 ml Benzol/Nitromethan 1:1 gelöst. Nach Abdestillieren von 250 ml Lösungsmittelmischung wurden 20,6 g (81,5 mMol) $Hg(CN)_2$ und 35,2 g (85,6 mMol) 2,3,4,6 - Tetra - O - acetyl - alpha - D - galactopyranosylbromid gelöst in 400 ml Benzol/Nitromethan 1:1 bei 60°C unter Rühren und Feuchtigkeitsausschluß langsam zugegeben. Nach 1,5 h Rühren wurden noch 1,7 g 2,3,4,6 - Tetra - O - acetylalpha - D - galactopyranosylbromid zugegeben. Nach 15 min war die alkoholische Komponente vollständig umgesetzt. Die Reaktionslösung wurde mit 500 ml Chloroform verdünnt, abfiltriert, zweimal mit 10 %iger wässriger Kaliumjodid-Lösung, dreimal mit gesättigter wässriger Natriumhydrogencarbonat-Lösung gewaschen, über Natriumsulfat getrocknet und zum Sirup (50 g) eingeengt. Für die Bestimmung von analytischen und spektroskopischen Daten wurde 1 g der resultierenden Rohverbindung chromatographisch ($CHCl_3$/Aceton 4:1; 25 g Kieselgel) gereinigt.

Ausbeute: 50 g (Rohprodukt) Sirup

$[\alpha]_D^{20}=2,49°$ (C=1 in $CHCl_3$)

$^1$H-NMR (270 MHz, $CD_3OD$) delta: H-1 4.62 d ppm ($J_{1,2}$=7.9 Hz); H-2 5.10 dd ($J_{2,3}$=10.5 Hz); H-3 3.4 dd; Ac 2.15 s, 2.04 s, 1.94 s.

| | |
|---|---|
| berechnet: | C 54,25 H 7,18 N 2,43 |
| gefunden: | C 54,22 H 7,15 N 2,39 |

Beispiel 2

N - (8 - Methoxycarbonylcapryl) - 2 - aminomethyl - 4,6 - O - benzyliden - beta - D - galactopyranosid (Verbindung 2)

50 g Verbindung 1 wurden in 200 ml trock. Methanol gelöst und mit 0,1 molarer methanolischen Natriummethylat-Lösung auf pH=11 eingestellt. Unter Feuchtigkeitsausschluß wurde die Lösung 8 h bei Raumtemperatur stehen gelassen. Die Reaktionsmischung wurde mit aktiviertem Ionenaustauscher Dowex® 50 WX neutralisiert und nach Abfiltrieren des Harzes zum Sirup eingeengt.

Das Rohprodukt (42 g) wurde mit 350 ml frisch destilliertem Benzaldehyd und 40 g trock. Zinkchlorid unter Feuchtigkeitsausschluß versetzt. Nach 12 h Rühren bei Raumtemperatur war die Umsetzung

vollständig (DC: Chloroform/Methanol 5:1). Das Reaktionsgemisch wurde mit einer wässrigen Natriumhydrogencarbonat-Lösung 10 min intensiv gerührt. Nach Abfiltrieren des anorganischen Niederschlages wurde das Filtrat mit Chloroform dreimal extrahiert. Die vereinigten anorganischen Phasen wurden über Natriumsulfat getrocknet und die Lösung wurde zuerst in Vakuum, dann in Hochvakuum zum Sirup eingeengt. Das Produkt kristallisierte in Acetonitril/Diisopropylether aus.

Ausbeute: 24 g (60%) $[\alpha]_D^{20}$=12,87°C
(c=1; Chloroform)

$^1$h-NMR (Die Verbindung 2 wurde, zum 2,3 - Di - O - acetyl - Derivat umgesetzt (Verbindung 4) und als solches charakterisiert.

| | |
|---|---|
| berechnet: | C 60,59 H 7,53 N 2,82 |
| gefunden: | C 60,90 H 7,51 N 2,80 |

Beispiel 3

N - (8 - Methoxycarbonylcapryl) - 2 - aminoethyl - 3 - O - acetyl - 4,6 - O - benzyliden - beta - D - galactopyranosid (Verbindung 3)

10 g (20 mMol) Verbindung 2 wurden in einer Mischung aus 500 ml trockenem Dichlormethan und 20 ml trockenem Pyridin gelöst. Bei −20°C wurden 1,73 g (22 mMol) Acet-ylchlorid gelöst in 10 ml trockenem Dichlormethan zugetropft. Nach 30 min war die Ausgangsverbindung vollständig umgesetzt (DC: Chloroform/Aceton 1:1). Nach Einengen der Reaktionsmischung wurde der resultierende Sirup in Chloroform aufgenommen, zweimal mit verdünntem HCl und anschließend mit Wasser gewaschen, über Natriumsulfat getrocknet und nach Abfiltrieren zum Sirup eingeengt. Das Rohprodukt wurde noch chromatographisch gereinigt (100 g Kieselgel; Elutionsmittel: $CHCl_3$/Aceton 1:1).

Ausbeute: 10 g (91%) Sirup
$[\alpha]_D^{20}$=40.0° (c=1; Chloroform)

$^1$H-NMR (400 MHz, $CDCl_3$) delta: H-1 4,33 d ppm ($J_{1,2}$=8 Hz); H-2 3,92 dd ($J_{2,3}$=10,5); H-3 4,77 dd ($J_{3,4}$ 4,2 Hz); Ac 2,05s

| | |
|---|---|
| berechnet: | C 60,32 H 7,31 N 2,61 |
| gefunden: | C 60,34 H 7,30 N 2,58 |

Beispiel 4

N - (0 - Methoxycarbonyl capryl) - 2 - aminoethyl - 2,3 - di - O - acetyl - 4,6 - O - benzyliden - beta - D - galactopyranosid (Verbindung 4)

1 g (2 mMol) Verbindung 2 wurden mit 0,346 g (4,4 mMol) Acetylchlorid wie in Beispiel 3 umgesetzt und aufgearbeitet.

Ausbeute: 1,1 g (95%) Sirup
$[\alpha]_D^{20}$=+32,58° (c=1; Chloroform)

$^1$H-NMR (100 Mhz, $CDCl_3$) delta: H-1 4,52 d ppm ($J_{1,2}$=3,8 Hz); Ac 2,04s, 2,05s

| | |
|---|---|
| berechnet: | C 60,09 H 7,13 N 2,42 |
| gefunden: | C 59,96 H 7,08 N 2,29 |

Beispiel 5

N - (8 - Methoxycarbonylcapryl) - 2 - aminoethyl 3 - O - benzoyl - 4,6 - O - benzyliden - beta - D - galactopyranosid (Verbindung 5)

5 g (10 mMol) Verbindung 2 wurden in einer Mischung aus 10 ml trockenem Acetonitril und 20 ml trockenem Pyridin gelöst. Unter Feuchtigkeitsausschluß wurden 1,32 g (10 mMol) Benzoylcyanid gelöst in 10 ml trockenem Acetonitril bei −5°C innerhalb von 30 min zugetropft. Nach 4 h Rühren bei −5°C wurde die Reaktionsmischung mit Methanol versetzt und eingeengt. Der resultierende Sirup wurde in Chloroform aufgenommen, zweimal mit verdünntem HCl, dann mit Wasser gewaschen, getrocknet und eingeengt. Das Rohprodukt (7,5 g) wurde noch chromatographisch (70 g Kieselgel $CHCl_3$/Aceton 4:1) gereinigt.

Ausbeute: 5,2 g (86%)
Schmelzpunkt=95°C
$[\alpha]_D^{20}$=+72,1° (c=1; Chloroform)

$^1$H-NMR (270 MHz, $CDCl_2$) delta: H-1 4,37 d ppm ($J_{1,2}$=7,9 Hz); H-2 4,07 dd ($J_{2,3}$=10,6 Hz); H-3 5,07 dd ($J_{3,4}$=3,6 Hz); PhCH 5,45s

| | |
|---|---|
| gerechnet: | C 64,09 H 6,89 N 2,34 |
| gefunden: | C 64,04 H 7,00 N 2,23 |

Beispiel 6

N - (8 - Methoxycarbonylcapryl) - 2 - aminoethyl - 2 - O - (2,3,4 - tri - O - benzyl - alpha - L - fucopyranosyl) - 4,6 - O - benzyliden - beta - D - galactopyranosid (Verbindung 6)

10 g (19 mMol) Verbindung 3, 4,8 g (19 mMol) Quecksilber (11)-cyanid, 40 g Molekularsieb 4 Å (Pulver) wurden zuerst mit Toluol destilliert und anschließend in 400 ml trockenem Dichlormethan bei Raumtemperatur unter Feuchtigkeitsausschluß suspendiert. Der Suspension wurden 11 g (22,8 mMol) frisch zubereitetes 2,3,4 - Tri - O - benzyl - alpha - L - fucopyranosylbromid, gelöst in 100 ml trockenem Dichlormethan innerhalb von 3 h zugetropft. Nach 24 h Rühren wurden weitere 2,2 g (4,5 mMol) 2,3,4 - Tri - O - benzyl - alpha - L - fucopyranosylbromid zugetropft. Die Umsetzung zum Disaccharid ist nach weiteren 6 h vollständig beendet (DC: Chloroform/Aceton 4:1). Die Suspension wurde über Celite filtriert, der Rest sorgfältig mit Dichlormethan gewaschen. Die vereinigten Filtrate wurden zweimal mit wässriger KH-Lösung, zweimal mit wässriger Natriumhydrogencarbonat-Lösung und einmal mit Wasser gewaschen. Nach dem Einengen wurde der resultierende Sirup noch mit Toluol destilliert.

Für analytische und spektroskopische Zwecke wurde eine kleine Menge des Rohproduktes säulenchromatisch gereinigt.

Zum Entacetylieren wurde das Rohprodukt (26 g) in Methanol gelöst und mit methanolischer Natriummethylatlösung versetzt. Nach der üblichen Aufarbeitung wurde das Produkt säulenchromatographisch gereinigt ($CHCl_3$/Aceton 4:1; 150 g Kieselgel).

Ausbeute: 14,5 g (81%) amorph

Schmelzpunkt:=126°C

$[\alpha]_D^{20}$=49,6° (c=1,5 in $CHCl_3$)

$^1$H-NMR (400 MHz, $CDCl_3$) delta: H-1 4,34 ppm ($J_{1,2}$=7,7 Hz); H-2 3,83 dd ($J_{2,3}$=9,5); H-3 3,78 dd; H-1' 5,09 d ($J_{1',2'}$=3,4 Hz) H-2' 4,07 dd ($J_{1',2'}$=10,2 Hz); H-6' 1,13 d ($J_{5',6'}$=6 Hz); PhCH 5,58s.

| | |
|---|---|
| berechnet: | C 68,48, H 7,18 N 1,54 |
| gefunden: | C 68,80 H 7,20 N 1,50 |

Beispiel 7

N - (8 - Methoxycarbonylcapryl) - 2 - aminoethyl - 3 - O - benzoyl - 2 - O - (2,3,4 - tri - O - benzyl - alpha - L - fucopyranosyl) - 4,6 - O - benzyliden - beta - D - galactopyranosid (Verbindung 7).

10 g (17 mMol) Verbindung 5 wurden in gleicher Weise zu einem Disaccharid umgesetzt, wie in Beispiel 6 beschrieben.

Ausbeute: 13,5 g (81%)

$[\alpha]_D^{20}$=−34,9° (C=1 in $CHCl_3$)

$^1$H-NMR (270 MHz, $CDCl_3$) delta: H-1 4,36 d ppm; H-3 5,34 ($J_{2,3}$=9,8 Hz; $J_{3,4}$=3,6 Hz); H-1' 5,24 ($J_{1',2'}$=3,3 Hz).

| | |
|---|---|
| berechnet: | C 69,74 H, 6,84 H 1,38 |
| gefunden: | C 69,62 H 6,82 N 1,31 |

Die Verbindung 7 wurde in gleicher Weise, wie in Beispiel 6 beschrieben, zur Verbindung 6 entacyliert.

Beispiel 8

N - (8 - Methoxycarbonylcapryl) - 2 - aminoethyl - 2 - O - (2,3,4 - tri - O - benzyl - alpha - L - fucopyranosyl) - 3 - O - (2,3,4,6 - tetra - O - benzyl - alpha - D - galactopyranosyl) - 4,6 - O - benzyliden - beta - D - galactopyranosid (Verbindung 8).

5 g (5,58 mMol) Verbindung 6, 14,1 g (55,8 mMol) Quecksilbercyanid, 2,0 g (5,58 mMol) Quecksilberbromid, 50 g Molekularsieb 0,4 nm (4 Å) und 10 g Drierite wurden mit Toluol destilliert und anschließend in 350 ml trockenem Dichlormethan suspendiert. Die Suspension wurde unter Feuchtigkeitsausschluß 1 h gerührt. Nach Zugabe von 9,6 g (16,74 mMol) 2,3,4,6 - Tetra - O - benzyl - alpha - D - galactopyranosylbromid, gelöst in 100 ml trockenem Dichlormethan, wurde die Suspension unter $N_2$-Atmosphäre und bei gleichzeitiger Ableitung von HCN 24 h gerührt. Es wurde mit $CH_2Cl_2$ filtriert und nachgewaschen. Die organische Phase wurde zweimal mit wässriger KJ-Lösung, dann mit wässriger Natriumhydrogencarbonat-Lösung und anschließend mit Wasser gewaschen und eingeengt. Der Sirup wurde chromatographisch gereinigt (400 g Kieselgel; Chloroform/Aceton 20:1 und Chloroform/Aceton/Methanol 4:1:1).

Ausbeute: 6,7 g (86%) Sirup

$[\alpha]_D^{20}$=−13,4° (C=1 in $CHCl_3$)

$^1$H-NMR (270 MHz, $CDCl_3$) delta: H-1, 4,64 d ppm ($J_{1,2}$=7,4 Hz); H-1' 5,32 d ($J_{1',2'}$=3,1 Hz); H-1'' 5,33 d ($J_{1'',2''}$=2,9 Hz) PhCH 5,53s.

| | |
|---|---|
| berechnet: | C 72,00 H 6,95 H 0,98 |
| gefunden: | C 71,73 H 6,96 N 0,93 |

Beispiel 9

N - (8 - Methoxycarbonylcapryl) - 2 - aminoethyl - 2 - O - (alpha - L - fucopyranosyl) - 3 - O - (alpha - D - galactopyranosyl) - beta - D - galactopyranosid (Verbindung 9).

6 g (4,4 mMol) Verbindung 8 wurden in 400 ml frisch destilliertem Eisessig gelöst und in Gegenwart von 6 g Palladiumkohle (10%) 20 h bei Raumtemperatur hydriert. Dann wurde filtriert, mit Eisessig nachgewaschen und im Vakuum eingeengt. Der Rückstand wurde in Methanol gelöst und über Celite filtriert. Das Produkt kristallisierte aus Ethanol/Essigester.

Ausbeute: 2,7 g (81%) amorph
$[\alpha]_D^{20}=-22{,}98°$ (c=1 $H_2O$)
Schmelzpunkt=170°C

$^1$H-NMR (400 MHz, $CDCl_3$) delta: H-1 4,57 d ppm ($J_{1,2}$=7,8 Hz); H-1' 5,28 d ($J_{1',2'}$=3,3 Hz); H-1'' 5,27 d ($J_{1'',2''}$=3,2 Hz).

| | |
|---|---|
| berechnet: | C 50,34 H 7,46 N 1,96 |
| gefunden: | C 49,90 H 7,31 N 1,91 |

Beispiel 10

N - (8 - Methoxycarbonylcapryl) - 2 - aminoethyl - 3 - O - (2 - azido - 2 - desoxy - alpha - D - galactopyranosyl) - 2 - O - (2,3,4 - tri - O - benzyl - alpha - D - fucopyranosyl) - 4,6 - benzyliden - beta - D - galactopyranosid (Verbindung 10).

7,47 g (8,3 mMol) Verbindung 6, 6,32 g (24,9 mMol) Quecksilbercyanid, 9,01 g (24,9 mMol) Quecksilberbromid und 50 g Molekularsiebe 0,4 nm (4 Å) wurden zuerst mit Toluol destilliert und dann in 40 ml trockenem Dichlormethan suspendiert. Die Suspension wurde unter Feuchtigkeitsausschluß 1 h gerührt. Nach Zugabe von 10 g (24,9 mMol) 3,4,6 - Tri - O - acetyl - 2 - azido - 2 - desoxy - alpha - D - pyranosylbromid, gelöst in 150 ml trockenem Dichlormethan, wurde die Suspension unter $N_2$-Atmosphäre und gleichzeitiger Ableitung der freiwerdenden HCN 48 h gerührt. Die Reaktionsmischung wurde mit 250 ml Dichlormethan versetzt, abfiltriert und das Filtrat wurde zweimal mit wäßriger KJ-Lösung, zweimal mit gesättigter Natriumhydrogencarbonat-Lösung und einmal mit Wasser gewaschen. Die organische Phase wurde mit Natriumsulfat getrocknet, im Vakuum eingeengt und mit Toluol destilliert. Der resultierende Sirup (13,3 g) wurde in trockenem Methanol gelöst und mit einmolarer methanolischer Natriummethylat-Lösung auf pH 11 eingestellt. Nach 8 h war die Entacetylierung beendet. Die Lösung wurde mit Eisessig neutralisiert und im Vakuum eingeengt. Der resultierende Sirup wurde in Chloroform aufgenommen und einmal mit Wasser gewaschen. Nach dem Einengen wurde das Produkt säulenchromatographisch gereinigt (400 g Kieselgel; Toluol/Aceton 1:1).

Ausbeute: 6,93 g (76%)
IR $cm^{-1}$ (2110, 1740, 1705, 1600, 1500)

| | |
|---|---|
| berechnet: | C 63,37 H 6,79 N 5,10 |
| gefunden: | C 63,29 H 6,80 N 5,2 |

0,5 g des Produktes wurden acetyliert und NMR-spektrometrisch als 3,4,6 - Tri - O - acetyl - Derivat charakterisiert.

$[\alpha]_D^{20}=+28{,}7°$ (c=1 in $CHCl_3$)

$^1$H-NMR (400 MHz, $CDCl_3$) delta: H-1 4,36 d ppm ($J_{1,2}$=7,6 Hz); H-2 3,95 dd ($J_{2,3}$=10,5 Hz); H-1' 5,12 d ($J_{1',2'}$=3,7 Hz); H-2' 4,03 dd ($J_{2',3'}$=11,0 Hz); H-1'' 5,33 d ($J_{1'',2''}$=3,5 Hz); H-2'' 2,55 dd ($J_{2'',3''}$=10,3 Hz).

Beispiel 11

N - (8 - Methoxycarbonylcapryl) - 2 - aminoethyl - 3 - O - (2 - acet - amido - 2 - desoxy - alpha - D - galactopyranosyl) - 2 - O - (alpha - L - fucopyranosyl) - beta - D - galactopyranosid (Verbindung 11).

7.0 g (5,6 mMol) Verbindung 10 wurden in 300 ml Eisessig gelöst und in Gegenwart von 12 g Palladiumkohle (10%) 4 h bei Raumtemperatur hydriert. Zur Aufarbeitung wurde mit 100 ml Eisessig verdünnt, filtriert, nachgewaschen und im Vakuum eingeengt. Der Rückstand wurde in 100 ml Methanol/Acetanhydrid 10:1 gelöst und im Vakuum langsam eingeengt. Das amorphe Produkt wurde in 150 ml Methanol gelöst und der Lösung wurden 40 ml konzentriertes Ammoniak zugegeben. Nach 24 h zeigte das Dünnschichtchromatogramm eine einheitliche Substanz ($CHCl_3$/$CH_3OH$/$H_2O$ 65:35:8). Nach Eindampfen im Vakuum wurde der Rückstand über Sephadex G-25 gereinigt.

Ausbeute: 3,15 (76%) amorph
Schmelzpunkt=190°C
$[\alpha]_D^{20}=+31{,}0°$ (C=1 in $H_2O$)
IR $cm^{-1}$ (1740, 1670)

$^1$H-NMR (400 MHz, $D_2O$) delta: H-1 4,57 d ppm ($J_{1,2}$=7,6 Hz); H-1' 5,28 d ($J_{1',2'}$=3,0 Hz); H-1'' 5,18 d ($J_{1'',2''}$=3,3 Hz); Ac 2,05 s

| | |
|---|---|
| berechnet: | C 50,79 H 7,46 N 3,70 |
| gefunden: | C 50,82 H 7,50 N 3,61 |

Beispiel 12

N - (8 - Methoxycarbonylcapryl) - 2 - aminomethyl - 3 - O - (3,4,6 - tri - O - acetyl - 2 - desoxy - 2 - jodo - alpha - D - talopyranosyl) - 3 - O - (2,3,4 - tri - O - benzyl - alpha - D - fucopyranosyl) - 4,6 - O - benzyliden - beta - D - galactopyranosid (Verbindung 12).

1 g (1,12 mMol) Verbindung 6 und 0,302 g (1,34 mMol) N-Jod-succinimid wurden zuerst mit Toluol destilliert und anschließend in 30 ml trockenem Aceton-itril gelöst. Unter Feuchtigkeitsausschluß wurden 0,34 g (1,23 mMol) 3,4,6 - Tri - O - acetyl - D - galactal, gelöst in 30 ml trockenem Acetonitril, zugegeben. Die Mischung wurde 8 h bei 60°C dann noch 50 h bei Raumtemperatur gerührt (DC: Toluol/Aceton 1:1). Die Lösung wurde mit Dichlormethan versetzt und mit Wasser gewaschen. Die organische Phase wurde im Vakuum eingeengt und der resultierende Sirup wurde chromatographisch gereinigt (30 g Kieselgel, Toluol/Aceton 2:1).

Ausbeute: 1,0 g (73%) Sirup

$[\alpha]_D^{20}=+53°$ (C=1 in $CHCl_3$)

$^1$H-NMR (400 MHz), $CDCl_3$) delta: H-1 4,41 d ppm ($J_{1,2}$=7,7 Hz); H-1' 5,29 d ($J_{1',2'}$=0,9 Hz); H-1'' 5,35 d ($J_{1'',2''}$=3,4 Hz)

| | |
|---|---|
| berechnet: | C 59,39 H 6,18 I 9,82 N 1,08 |
| gefunden: | C 59,03 H 6,20 I 9,37 N 1,02 |

Beispiel 13

N - (8 - Methoxycarbonyl) - 2 - aminomethyl - 3 - O - (2 - desoxy - alpha - D - galactopyranosyl) - 2 - O - (alpha - L - fucopyranosyl) - D - galactopyranosid (Verbindung 13).

1 g (0,77 mMol) Verbindung 12 wurden in 80 ml Eisessig gelöst und in Gegenwart von 1,6 g Palladiumkohle (10%) 72 h bei Raumtemperatur hydriert. Zur Aufarbeilung wurde mit 50 ml Eisessig verdünnt, filtriert, nachgewaschen und im Vakuum eingeengt. Der Rückstand wurde mit Toluol destilliert und anschließend wurde der Sirup in einer Mischung aus 20 ml Methanol und 6 ml konzentriertem Ammoniak gelöst. Nach 24 h zeigte das Dünnschichtchromatogramm eine einheitliche Substanz ($CHCl_3$ /Methanol/Wasser 65:35:5). Nach Eindampfen im Vakuum wurde der Rückstand in Methanol/Aceton kristallisiert.

Ausbeute: 0,38 g (73%)

$[\alpha]_D^{20}=+53,8°$ (C=1 in $H_2O$)

$^1$H-NMR (400 MHz, $D_2O$) delta: H-1 4,46 d ppm ($J_{1,2}$=7,7 Hz); H-1' 4,67 dd ($J_{1',2'a}$=1,0 Hz, $J_{1'2'e}$=3,6 Hz); H-1'' 5,21 d ($J_{1'',2''}$=3,2 Hz)

IR $cm^{-1}$ (1740, 1660)

| | |
|---|---|
| berechnet: | C 52,70 H 7,75 N 2,05 |
| gefunden: | C 52,81 H 7,73 N 2,01 |

Beispiel 14

Inhibition von humanem Anti-A mit Verbindung 11 und humanem Anti-B mit Verbindung 9

Die Hämagglutination von 5 %iger A-Erythrozyten-Suspension mit humanem Anti-A (Titer $2^{-5}$) wurde vollständig mit Verbindung 11 (0,5 mg/ml) inhibiert. Die Verbindung 9 zeigte keine inhibition.

Die Hämagglutination von 5 %iger B-Erythrozyten-Suspension mit humanem Anti-B (Titer $2^{-5}$) wurde vollständig mit Verbindung 9 (0,3 mg/ml) inhibiert. Die Verbindung 11 zeigte keine Inhibition.

Beispiel 15

N - [8 - (2 - Aminoethylcarbamoyl) - capryl] - 2 - aminoethyl - 3 - O - (2 - acetamido - 2 - desoxy - alpha - D - galactopyranosyl) - 2 - O - (alpha - D - fucopyranosyl) - beta - D - galactopyranosid (Verbindung 15).

100 mg (0,13 mMol) Verbindung 11 wurden in 20 ml 85 %igen wässrigem 1,2-Diaminoethan gelöst und 24 h bei Raumtemperatur gerührt (DC: $CHCl_3$ /Methanol/konz. $NH_3$ 2:2:1). Die Mischung wurde gefriergetrocknet und der amorphe Rest wurde noch mehrere Male mit Methanol/Toluol destilliert. Die Verbindung 15 wurde dünnschichtchromatographisch ($CHCl_3$ /Methanol/konz. $NH_3$ 1:2:1, Sprühreagenz Ninhydrin) nachgewiesen.

Ausbeute: 100 mg

IR (2 mg/100 mg KBr) $cm^{-1}$ (3500—3200, 1640)

Wie in Beispiel 15 beschrieben, wurden folgende Verbindungen hergestellt und dünnschicht-chromatographisch und IR-spektroskopisch charakterisiert.

N - (8 - (2 - Aminoethylcarbamoyl) - capryl) - 2 - aminoethyi - beta - D - galactopyranosid (Verbindung 16)

N - (8 - (2 - Aminoethylcarbamoyl) - capryl) - 2 - aminoethyl - 2 - O - (alpha - L - fucopyranosyl) - beta - D - galactopyranosid (Verbindung 17)

N - (8 - (2 - Aminoethylcarbamoyl) - capryl) - 2 - aminoethyl - 3 - O - (alpha - D - galactopyranosyl) - 2 - O - (alpha - D - fucopyranosyl) - beta - D - galactopyranosid (Verbindung 18)

N - (8 - (2 - Aminoethylcarbamoyl) - capryl) - 2 - aminoethyl - 3 - O - (2 - desoxy - alpha - D - galactopyranosyl) - 2 - O - (alpha - L - fucopyranosyl) - beta - D - galactopyranosid (Verbindung 19)

Beispiel 16

N - (8 - Hydrazinocarbonylcapryl) - 2 - aminoethyl - 3 - O - (alpha - D - galactopyranosyl) - 2 - O - (alpha - D - fucopyranosyl) - beta - D - galactopyranosid (Verbindung 20)

100 mg (140 µmol) Verbindung 9 wurden in 5 ml 80 %igen wässrigen Hydrazinhydrat 24 h bei Raumtemperatur gerührt (DC: Essigester/Methanol/Wasser 5:3:3). Die Lösung wurde gefriergetrocknet und anschließend wurde der amorphe Rest mit Methanol/Toluol mehrmals destilliert.

Ausbeute: 100 mg

IR (2 mg Probe/100 mg KBr) $cm^{-1}$ (3500—3200, 1680, 1640).

Wie in Beispiel 16 beschrieben, wurden folgende Verbindungen hergestellt und dünnschichtchromatographisch und IR-spektroskopisch charakterisiert:

N - (8 - Hydrazinocarbonylcapryl) - 2 - aminoethyl - beta - D - galactopyranosid (Verbindung 21)

N - (8 - Hydrazinocarbonylcapryl) - 2 - aminoethyl - 2 - O - (alpha - L - fucopyranosyl) - beta - D - galactopyranosid (Verbindung 22)

N - (8 - Hydrazinocarbonylcapryl) - 2 - aminoethyl - 3 - O - (2 - acetamido - 2 - desoxy - alpha - D - galactopyranosyl) - 2 - O - (alpha - L - fucopyranosyl) - beta - D - galactopyranosid (Verbindung 23)

N - (8 - Hydrazinocarbonylcapryl) - 2 - aminoethyl - 3 - O - (2 - desoxy - alpha - D - galactopyranosyl) - 2 - O - (alpha - L - fucopyranosyl) - beta - D - galactopyranosid (Verbindung 24)

Beispiel 17

N - (8 - Azidocarbonylcapryl) - 2 - aminoethyl - 3 - O - (alpha - D - galactopyranosyl) - 2 - O - (alpha - L - fucopyranosyl) - beta - D - galactopyranosid (Verbindung 25)

200 mg (280 µMol) Verbindung 20 wurden in 2 ml trockenem DMF gelöst und auf −26°C abgekühlt. Eine Lösung von 0,3 ml trockenem Dioxan, die 3,5 N Chlorwasserstoffsäure enthält, wurde zugegeben und dann wurden 45,2 mg (380 µMol) iso-Pentylnitrit, gelöst in 1 ml trockenem DMF, zugegeben. Dieses Gemisch wurde 30 min bei −25°C gerührt, danach wurden 28 mg (300 µMol) Amidoschwefelsäure zugegeben (DC: Essigester/Methanol/Wasser 5:3:3).

Die Reaktion wurde dünnschichtchromatographisch verfolgt (Rf=0,45). Die Lösung, die das Carbonsäureazid (Verbindung 25) enthält, wurde ohne weitere Reinigungsschritte für die Umsetzung mit aminierten Trägern verwendet.

Wie in Beispiel 17 beschrieben, wurden folgende Verbindung hergestellt und dünnschichtchromatographisch charakterisiert:

N - (8 - Azidocarbonylcapryl) - 2 - aminoethyl - beta - D - galactopyranosid (Verbindung 26)

N - (8 - Azidocarbonylcapryl) - 2 - aminoethyl - 2 - O - (alpha - L - fucopyranosyl) - beta - D - galactopyranosid (Verbindung 27)

N - (8 - Azidocarbonylcapryl) - 2 - aminoethyl - 3 - O - (2 - acetamido - 2 - desoxy - alpha - D - galactopyranosyl) - 2 - O - (alpha - L - fucopyranosyl) - beta - D - galactopyranosid (Verbindung 28)

N - (8 - Azidocarbonylcapryl) - 2 - aminoethyl - 3 - O - (2 - desoxy - alpha - D - galactopyranosyl) - 2 - O - (alpha - L - fucopyranosyl) - beta - D - galactopyranosid (Verbindung 29)

Beispiel 18

N - (8 - Hydroxycarbonylcapryl) - 2 - aminoethyl - 2 - O - (alpha - L - fucopyranosyl) - 3 - O - (alpha - D - galactopyranosyl) - beta - D - galactopyranosid (Verbindung 30)

100 mg (140 µMol) Verbindung 9 wurden mit 4 ml 0,1 N wässrigem NaOH bei Raumtemperatur behandelt. (DC=Dünnschichtchromatografie: Essigester/Methanol/$H_2O$ 5:3:3). Nach der Deionisierung mit Ionenaustauscher wurde die Probe lyophilisiert. Ausbeute: 100 mg.

Wie im Beispiel 18 beschrieben, wurden folgende Verbindungen hergestellt und dünnschichtchromatographisch charakterisiert:

N - (8 - Hydroxycarbonylcapryl) - 2 - aminoethyl - 2 - O - beta - D - galactopyranosid (Verbindung 31)

N - (8 - Hydroxycarbonylcapryl) - 2 - aminoethyl - 2 - O - (alpha - L - fucopyranosyl) - beta - D - galactopyranosid (Verbindung 32)

N - (8 - Hydroxycarbonylcapryl) - 2 - aminoethyl - 3 - O - (2 - acetamido - 2 - desoxy - alpha - D - galactopyranosyl) - 2 - O - (alpha - L - fucopyranosyl) - beta - D - galactopyranosid (Verbindung 33)

N - (8 - Hydroxycarbonylcapryl) - 2 - aminoethyl - 3 - O - (2 - desoxy - alpha - D - galactopyranosyl) - 2 - O - (alpha - L - fucopyranosyl) - beta - D - galactopyranosid (Verbindung 34)

Die Verbindungen 30 bis 34 wurden entweder direkt mit Carbodiimid (zum Beispiel 1 - Ethyl - 3 - (3' - dimethylamino - propyl) - carbodiimid - Hydrochlorid oder als aktivierte Ester (zum Beispiel N-Hydroxysuccinimid-Derivate) an proteine (Humanserumalbumin) oder Polypeptide (Polylysin) nach bekannten Verfahren gekoppelt.

Beispiel 19

Immobilisierung von aminierten Haptenen (Verbindungen 15 bis 19) an Oxirangruppen tragendes Träger-Eupergit C oder mit Epichlorhydrin-aktiviertem Magnogel

5 g Eupergit® C (Firma Röhm Pharma) wurden in Phosphatpuffer pH 7,2 (50 ml) suspendiert und 16 h bei Raumtemperatur stehen gelassen. Der Träger wurde mit $CO_2$-freiem Wasser, dann mit 1/15 molarem wässrigen $Na_2HPO_4$ und $Na_2B_4O_7$-Lösung (pH 9,2) gewaschen und das Volumen auf 50 ml eingestellt. Zu dieser Suspension wurden 50 µMol einer aminierten Verbindung zugegeben und es wurde 24 h bei Raumtemperatur geschüttelt. Zur Umsetzung der restlichen Oxirangruppen des Trägers wurden noch 5 ml einer 10 %igen wässrigen Ethanolamin-Lösung zugegeben und die Suspension wurde erneut 48 h geschüttelt.

Das Immunadsorbens wurde mit Wasser, dann mit Phosphatpuffer gewaschen. Das feuchte Gel wurde bei 4°C gelagert.

Analytische Bestimmung der D-Galactose beziehungsweise L-Fucose im Immunoadsorbens:

| | µMol Zucker/g Träger | | | |
|---|---|---|---|---|
| Immobilisierte | Gal | | Fuc | |
| Verbindung | gerechnet | gefunden | gerechnet | gefunden |
| 15 | 10 | 10 | 10 | 9,3 |
| 16 | 10 | 9,6 | — | — |
| 17 | 10 | 9,8 | 10 | 10 |
| 18 | 20 | 19,4 | 10 | 10 |
| 19 | 10 | 9,6 | 10 | 9,8 |

Beispiel 20

Immobilisierung von Carbonylazid-derivatisierten Haptenen (Verbindungen 25 bis 29) an aminierte Träger

5 g aminiertes Eupergit C (Herstellungsverfahren siehe unten) wurden in 18 ml 0,08 M $Na_2B_4O_7$— und 0,35 M $KHCO_3$-Puffer suspendiert. Das in situ hergestellte Carbonylazid-Derivat (Verbindungen 25 bis 29) wurde bei 0°C zu der Suspension zugetropft und anschließend wurde noch 24 h bei 4°C geschüttelt. Das Immunoadsorbens wurde mit $CO_2$-freiem Wasser gewaschen, dann in 18 ml wässriger gesättigter $NaHCO_3$-Lösung aufgenommen und mit 18 ml 5 %igem Acetanhydrid versetzt. Nach 15 min Schütteln wurde das Adsorbens abfiltriert, mit Wasser, dann mit 2 %iger wässriger Amoniak-Lösung und schließlich mit Wasser gewaschen. Das feuchte Immunoadsorbens wurde bei 4°C gelagert.

Aminierung von Oxiran-aktivierten Trägern

50 g Oxiran-aktivierte Träger (bevorzugt Eupergit® C oder Magnogel® mit Epichlorhydrien aktiviert) wurden in 500 ml $CO_2$-freiem destilliertem Wasser 2 h suspendiert. Nach der Zugabe von 27,5 ml (0,63 Mol) 1,2-Diaminoethan wurde die Suspension 1 b bei Raumtemperatur gerührt. Nach Abfiltrieren und Waschen mit destilliertem Wasser und mit Ethanol wurde der Träger 1 h bei 40°C getrocknet. Nach diesem Verfahren weist 1 g Träger minimal 1,2 mMol primäre Aminogruppen auf.

Analytische Bestimmung der D-Galactose beziehungsweise L-Fucose in Immunoadsorbens.

| | µMol Zucker/g Träger | | | |
|---|---|---|---|---|
| Immobilisierte | Gal | | Fuc | |
| Verbindung | gerechnet | gefunden | gerechnet | gefunden |
| 25 | 20 | 20 | 10 | 10 |
| 26 | 10 | 9,8 | — | — |
| 27 | 10 | 9,9 | 10 | 9,6 |
| 28 | 10 | 9,4 | 10 | 9,6 |
| 29 | 10 | 9,7 | 10 | 9,7 |

Beispiel 21

Immunadsorption von Anti-A-Serum an Blutgruppen A-aktivem Immunadsorbens

5 g Immunoadsorbens A (Beispiel 20, Verbindung 28) wurden in 100 ml 0,9% NaCl-Lösung suspendiert, in eine kleine Chromatographiesäule hineingegeben und mit 150 ml 0,9% NaCl-Lösung gewaschen. 100 ml Serum (mit Anti-A-Titer 1:64) wurden chromatographiert. Das Immunadsorbens wurde mit NaCl-Lösung gewaschen und anschließend wurden die adsorbierten Anti-A-Antikörper mit 20 ml Elutionsmittel (1% wässriges Ammoniak, 0,1 Mol N-Acetylaminoethanol und 0,9 Mol Ethylenglycol) desorbiert. Nach Auswaschen des Eluationsmittels wurde der Träger mit 150 ml NaCl-Lösung gewaschen und ist somit für die nächste chromatographische Trennung wider verwendbar.

In gleicher Weise wurden die Seren mit Anti-B an Immunadsorbens B (Beispiel 20, Verbindung 25) chromatographiert.

Die chromatographierten Seren zeigten bei der serologischen Untersuchung keinen Anti-A-beziehungsweise Anti-B-Titer.

Bei der Anwendung von Immunadsorbens A beziehungsweise B im "Batch-Verfahren" konnte der Antikörpertiter (Anti-A beziehungsweise Anti-B) wesentlich reduziert werden.

**Patentansprüche**

1. Verbindung der allgemeinen Formel I

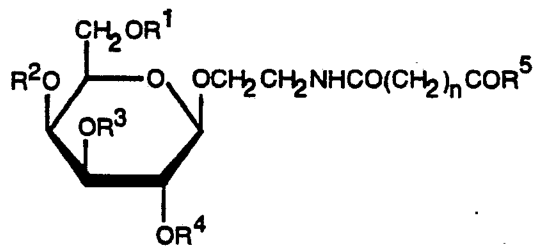

I

worin

$R^1$ und $R^2$ je ein Wasserstoffatom, eine Acyl- oder Benzyl-Schutzgruppe,

$R^1$ und $R^2$ zusammen eine Alkyliden- oder Benzyliden-Schutzgruppe,

$R^4$ ein Wasserstoffatom, eine Acyl- oder Benzyl-Schutzgruppe oder einen 2,3,4 - Tri - O - benzyl - alpha - L - fucopyranosyl - oder alpha - L - Fucopyranosyl - Rest,

$R^5$ H, OH, $NH_2$, $NHNH_2$, $N_3$, O-Alkyl, O-Aryl, NH—$(CH_2)_mNH_2$ mit m=2—5, Lysin, Polylysin oder einen Träger, n 1 bis 10 und

$R^3$ ein Wasserstoffatom, eine Acyl- oder Benzyl-Schutzgruppe oder einen alpha - D - Glycopyranosyl - Rest der allgemeinen Formel II bedeuten,

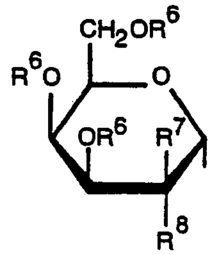

II

worin

$R^6$ ein Wasserstoffatom oder eine Acyl- oder Benzyl-Schutzgruppe,

$R^7$ ein Wasserstoffatom oder Halogen und

$R^8$ ein Wasserstoffatom, eine Acyloxy- oder Benzyloxy-Gruppe oder eine Azido-, Amino-, Acetamido- oder Hydroxygruppe bedeuten.

2. Verbindung der Formel I, worin der Kohlenhydratrest ß-D-Galactopyranosyl, oder $R^1$ und $R^2$ Wasserstoffatome und $R^4$ alpha-L-Fucopyranosyl, und

$R^3$ Wasserstoff, N - Acetyl - alpha - D - galactosaminyl, alpha - D - Galactopyranosyl oder 2 - Desoxy - alpha - D - galactopyranosyl und

$R^5$ $OCH_3$, $NHNH_2$, $N_3$, NH—$CH_2CH_2$, Protein, besonders Albumin, ein $NH_2$-Gruppen tragendes Gel oder Kephalin und n=7 sind.

3. Verfahren zur Herstellung einer Verbindung nach Anspruch 1, dadurch gekennzeichnet, daß man eine Verbindung der allgemeinen Formel III

$$HOCH_2CH_2NHCO(CH_2)_nCOR^5 \qquad III$$

in der $R^5$ O-Alkyl oder O-Benzyl und n=1 bis 10 bedeuten, mit einer Verbindung der allgemeinen Formel IV

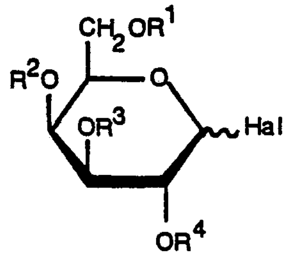

IV

in der $R^1$, $R^2$, $R^3$ und $R^4$ eine Acylgruppe, bevorzugt Acetyl- oder Benzoyl-Gruppe, und Hal Cl, Br oder J bedeuten, bevorzugt zu einem ß-Glycosid der Formel I umsetzt.

4. Verwendung einer Verbindung nach Anspruch 1 zur Bindung an einen Träger.

5. Verwendung einer Verbindung nach Anspruch 1 gebunden an einen Träger als Immunadsorbens.

6. Verwendung einer Verbindung der Formel I aus Anspruch 1, worin

$R^4$ alpha-L-fucopyranosyl und

$R^3$ Wasserstoff, N - Acetyl - alpha - D - galactosaminyl, alpha - D - Galactopyranosyl oder 2 - Desoxy - alpha - D - galactopyranosyl sind, gebunden an einen Träger als Immunadsorbens.

**Claims**

1. A compound of the formula I

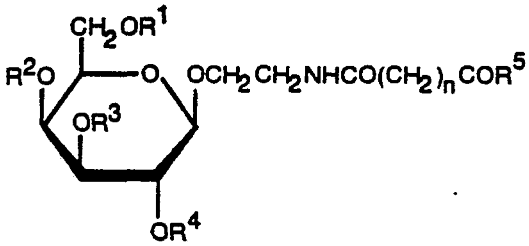

I

in which

$R^1$ and $R^2$ each denote a hydrogen atom or an acyl or benzyl protective group,

$R^1$ and $R^2$ together denote an alkylidene or benzylidene protective group,

$R^4$ denotes a hydrogen atom, an acyl or benzyl protective group or a 2,3,4 - tri - O - benzyl - α - L - fucopyranosyl or α-L-fucopyranosyl radical,

$R^5$ denotes H, OH, $NH_2$, $NHNH_2$, $N_3$, O-alkyl, O-aryl, $NH—(CH_2)_mNH_2$, where m=2—5, lysine, polylysine or a carrier,

n denotes a number from 1 to 10 and

$R^3$ denotes a hydrogen atom, an acyl or benzyl protective group or an α-D-glycopyranosyl radical of the general formula II,

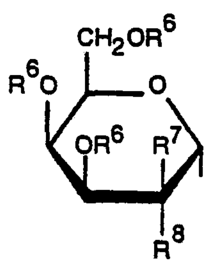

II

in which

$R^6$ denotes a hydrogen atom or an acyl or benzyl protective group,

$R^7$ denotes a hydrogen atom or halogen and

$R^8$ denotes a hydrogen atom, an acyloxy or benzyloxy group or an azido, amino, acetamido or hydroxyl group.

2. A compound of the formula I, in which the carbohydrate radical is ß-D-galactopyranosyl, or $R^1$ and $R^2$ are hydrogen atoms, $R^4$ is α-L-fucopyranosyl,

$R^3$ is hydrogen, N - acetyl - α - D - galactosaminyl, α-D-galactopyranosyl or 2 - deoxy - α - D - galactopyranosyl,

$R^5$ is $OCH_3$, $NHNH_2$, $N_3$, $NH—CH_2CH_2$, a protein, in particular albumin, a gel carrying $NH_2$ groups or cephalin and n=7.

3. Process for the preparation of a compound as claimed in claim 1, which comprises reacting a compound of the general formula III

$$HOCH_2CH_2NHCO(CH_2)_nCOR^5 \qquad III$$

in which $R^5$ denotes O-alkyl or O-benzyl and n is a number from 1 to 10, with a compound of the general formula IV

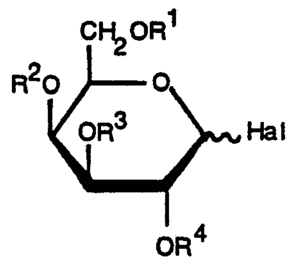

IV

in which $R^1$, $R^2$, $R^3$ and $R^4$ denote acyl groups, preferably acetyl or benzoyl groups, and Hal denotes Cl, Br or I, preferably to give a ß-glycoside of the formula I.

4. Use of a compound as claimed in claim 1 for bonding to a carrier.

5. Use of a compound as claimed in claim 1, bonded to a carrier, as an immuno-adsorbent.

6. Use of a compound of the formula I from claim 1, in which $R^4$ is α-L-fucopyranosyl and $R^3$ is hydrogen, N - acetyl - α - D - galactosaminyl, α-D-galactopyranosyl or 2 - deoxy - α - D - galactopyranosyl, bonded to a carrier, as an immuno-adsorbent.

**Revendications**

1. Composé de formule générale I

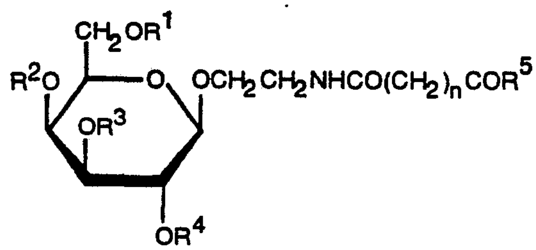

I

dans laquelle

$R^1$ et $R^2$ représentent chacun un atome d'hydrogène, un groupe protecteur acyle ou benzyle,

$R^1$ et $R^2$ représentent ensemble un groupe protecteur alcoylidène ou benzylidène,

$R^4$ représente un atome d'hydrogène, un groupe protecteur acyle ou benzyle ou un radical 2,3,4 - tri - O - benzyl - alpha - L - fucopyranosyle ou L-fucopyranosyle,

$R^5$ représente H, OH, $NH_2$, $NHNH_2$, $N_3$, un groupe O-alcoyle, O-aryle, $NH—(CH_2)_mNH_2$ avec m=de 2 à 5, la lysine ou la polylysine ou un support,

n est compris entre 1 et 10, et

$R^3$ représente un atome d'hydrogène, un groupe protecteur acyle ou benzyle ou un radical alpha - D - glycopyranosyle de formule générale II

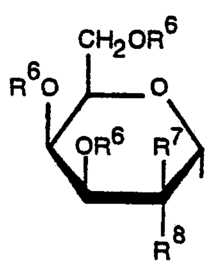

II

dans laquelle

$R^6$ représente un atome d'hydrogène ou un groupe protecteur acyle ou benzyle,

$R^7$ représente un atome d'hydrogène ou un halogène et

$R^8$ représente un atome d'hydrogène, un groupe acyloxy ou benzyloxy ou un groupe azido, amino, acétamido, ou hydroxy.

2. Composé de formule I, dans lequel le radical hydrate de carbone est le radical β-D-galactopyranosyle, ou

$R^1$ et $R^2$ sont des atomes d'hydrogène et $R^4$ est le radical alpha-L-fucopyranosyle, et $R^3$ est l'hydrogène, un radical N - acétyl - alpha - D - galactosaminyle, alpha-D-galactopyranosyle ou 2 - désoxy - alpha - D - galactopyranosyle et

$R^5$ est $OCH_3$, $NHNH_2$, $N_3$, $NH—CH_2CH_2$, une protéine, en particulier l'albumine, un gel portant des groupes $NH_2$ ou la céphaline, et n=7.

3. Procédé pour la préparation d'un composé selon la revendication 1, caractérisé en ce qu'on fait réagir un composé de formule générale III

$$HOCH_2CH_2NHCO(CH_2)_nCOR^5 \qquad III$$

dans laquelle $R^5$ est un groupe O-alcoyle ou O-benzyle et n est compris entre 1 et 10, avec un composé de formule générale IV

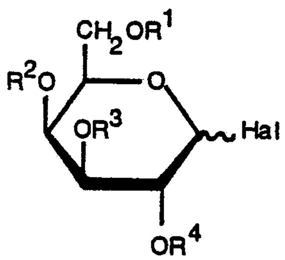

IV

dans laquelle

$R^1$, $R^2$, $R^3$ et $R^4$ représentent un groupe acyle, de préférence un groupe acétyle ou benzoyle, et Hal représente Cl, Br ou I, pour obtenir de préférence un β-glycoside de formule I.

4. Utilisation d'un composé selon la revendication 1 pour une fixation sur un support.

5. Utilisation d'un composé selon la revendication 1, fixé sur un support, en tant qu'immunoadsorbant.

6. Utilisation d'un composé de formule I selon la revendication 1, dans lequel

$R^4$ est un radical alpha-L-fucopyranosyle et

$R^3$ est l'hydrogène, un radical N - acétyl - alpha - D - galactosaminyle, alpha-D-galactopyranosyle ou 2 - désoxy - alpha - D - galactopyranosyle, fixé sur un support, en tant qu'immunoadsorbant.